# EUROPEAN PATENT APPLICATION

(11) **EP 1 216 995 A2**
(43) Date of publication of application: **26.06.2002**
(21) Application number: 01120993.9
(22) Date of filing: 31.08.2001
(51) Int. Cl.: C07D 209/86, C07C 15/46, C08F 12/32, C08F 112/32, C08F 297/02

(54) **Method for anionically polymerizing styrene derivatives containing carbazole as functional side group**

(30) Priority: 19.12.2000 KR 2000078168
(71) Applicant: Kwangju Institute of Science and Technology, Kwangyeok-si (KR)
(72) Inventor: Lee, Jae Suk, Buk-ku, Kwangju (KR); Cho, Young Sun, Buk-ku, Kwangju (KR); Ihn, Chi Sung, Buk-ku, Kwangju (KR); Lee, Hye Kyung, Buk-ku, Kwangju (KR)
(74) Representative: von Füner, Nicolai, Dr.

(57) **Abstract**

Disclosed are styrene derivatives containing carbazole as functional side group and their anionic polymerization. Styrene derivatives containing carbazole as functional side group, and homopolymers or copolymers of the styrene derivatives can be synthesized by the anionic polymerization method. Thusly synthesized high molecular weight polymer containing carbazole has advantages of thermal stability, optical properties, and defined molecular weight and limited molecular weight distribution.

## Description

### BACKGROUND OF THE INVENTION

### Field of the Invention

The present invention relates to styrene derivatives containing carbazole as functional side group and their anionic polymerization. More specifically, the present invention relates to a method for anionically polymerizing styrene derivatives containing carbazole as functional side group, and homopolymers or copolymers of, the styrene derivatives, by which these polymers can be provided with excellent thermal stability, optical properties, including photoluminescence and photoconductivity, and defined molecular weight and narrow molecular weight distribution.

### Description of the Prior Art

Carbazole-containing materials have been widely used as photoconductive, refractive and non-linear optical materials, and have been recently reported to be useful as media of photoluminescence materials [(1) Y. Zhang, T. Wada, and H.Sasabe, *Chem. Commun.,* **1996,** 621. (2) Y. Zhang, T. Wada, and H.Sasabe, *Macromol. Chem. Phys.,* **1996,** *197,* 667. (3) K. Tamura, A. B. Padias, H. K. Hall Jr., and N. Peyghambarian, *Appl. Phys. Lett*., **1992,** *60*, 1803.]. In addition, it is reported that carbazole-containing materials, together with associated metals, have been used as high molecular weight polymer for electrophotoluminescence [A. Ribou, T. Wada, and H. Sasabe, *Inorganica Chemica Acta*, **1999,** *288*, 134]. For the photorefractive, low glass transition temperature materials are required, while for the electrophotoluminescent, high glass transition temperature materials are needed because of thermal stability. Accordingly, if glass transition temperature can be controlled by the molecular weight of the polymer, the polymer can be used for various applications.

Since anionic polymerization of styrene has been found researches have been actively carried out. Recently, anionic polymerization techniques have been developed so that styrene can be prepared in large scale through the polymerization at plants. However, in the case of styrene with functional groups, undesirable side-reactions occur during the polymerization, and so studies for alleviating these problems have been performed throughout the world. In this regard, it is reported that, in styrene derivatives with functional electron donating groups to the para-position, side-reactions are caused so that molecular weight of the polymer and molecular weight distribution cannot be controlled properly[T. Ishizone, T. Utake, Y. Ishino, A. Hirao, and S. Nakahama, *Macromolecules*, **1987,** *30*, 6548]. Furthermore, when methylene group is introduced into styrene as a functional group, highly reactive growing chain end attacks methylene group during polymerization, whereby the polymers having defined molecular weight and narrow molecular weight distribution are difficult to obtain.

An effort has been directed toward successfully synthesizing high molecular weight polymer by introducing a functional group-protecting group to the polymerization reaction, and then removing the protecting group after the polymerization [T. Ishizone, G. Uehara, A. Hirao, S. Nakahama, and K. Tsuda, *Macromolecules*, **1998,** *31*, 3764].

When the monomer is a liquid, pure monomer can be obtained by distillation or other processes, while monomer of solid state has some impurities because distillation cannot be performed. Hence, such impurities reduce activity of initiator so that synthesized high molecular weight polymer suffers from the disadvantages of undesirable molecular weight and broadened molecular weight distribution.

### SUMMARY OF THE INVENTION

Accordingly, it is an object of the present invention to provide a solid styrene monomer containing carbazole as functional side group, which does not cause side-reactions in the anion polymerization thereof, unlike a methylene group-containing styrene derivative.

It is another object of the present invention to provide a homopolymer with a definite molecular weight and narrow molecular weight distribution, synthesized from the carbazole-containing solid styrene monomer at such a lowered reaction temperature as to restrain side-reactions.

It is a further object of the present invention to provide a method for synthesizing the homopolymer.

It is a still further object of the present invention to provide a block copolymer with defined molecular weight and limited molecular weight distribution, resulting from the block-polymerization of the carbazole-containing solid styrene monomer and other monomers at such a lowered reaction temperature as to restrain side-reactions.

It is a still further object of the present invention to provide a method for synthesizing the block copolymer.

### BRIEF DESCRIPTION OF THE DRAWINGS

The above and other objects, features and other advantages of the present invention will be more clearly understood from the following detailed description taken in conjunction with the accompanying drawings, in which:
FIG. 1 shows a mechanism for undesirable side-reactions during the polymerization used in the present invention.
FIG. 2 shows a reaction scheme for synthesizing the carbazole-containing monomer of the present invention.
FIG. 3 shows a reaction scheme of anionic polymerization used in the present invention.
FIG. 4 shows a TGA curve of thermal properties of synthesized polymer.
FIG. 5 shows DSC curves of thermal properties of synthesized polymer.
FIG. 6 shows spectra of photoluminescence properties of homopolymer synthesized in the present invention.

### DETAILED DESCRIPTION OF THE INVENTION

Based on the present invention, a 4-methylcarbazollyl styrene monomer containing carbazole as functional side group, a homopolymer with controlled molecular weight and narrow molecular weight distribution of the monomer, and a copolymer with controlled molecular weight and narrow molecular weight distribution of the monomer and styrene, methylmethacylate (MMA) or 9-ethylcarbazollyl methacrylate (CzMA) are synthesized.

A detailed description will be given of the present invention below.

### Synthesis of Monomer Containing Carbazole As Functional Side Group

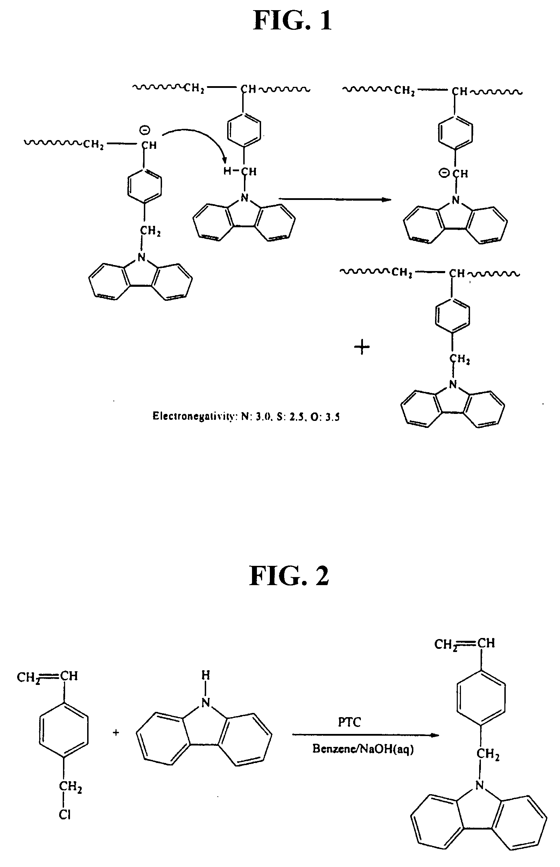

Carbazole was reacted with 1.5-fold excess of 4-vinylbenzyl chloride in benzene/water using NaOH as a base, and benzyltriethylammonium chloride as phase catalyst to afford 4-(9-carbazolyl)methylstyrene (CMS) at 80 °C for 5 hours. The reaction mixture was extracted with chloroform and the organic layer washed with water, evaporated, and washed with hexane several times. The solid was dissolved in and recrystallized from diethyl ether at -20 °C to yield a pure monomer, 4-methylcarbazollyl styrene (CMS). This reaction scheme is schematically shown in Fig. 2.

The identity and purity of the monomer is confirmed by use of ¹H-NMR, ¹³C-NMR and FT-IR. Undesirable side-reaction of the monomer prepared is seen in Fig. 1.

### Synthesis of Poly(4-(9-carbazollylmethyl styrene))

From the monomer obtained above, poly(4-(9-carbazollylmethyl styrene)) homopolymer is synthesized as follows.

Anionic polymerizations were carried out in THF under high-vacuum conditions (10⁻⁶ mmHg) for 5 minutes to 24 hours in an all-glass apparatus equipped with break-seals in the usual manner. For the homopolymerization of CMS, a THF solution of the CMS was added to K-Naph. solution in THF at -45 or -78 °C and allowed to react for 5 minutes to 24 hours, and then terminated with methanol. The polymers were precipitated in large amount of methanol, dried, dissolved in benzene, and freeze-dried. The yield of polymer was determined from ¹H-NMR data.

The synthesized polymer is then measured for its molecular weight and molecular weight distribution by use of GPC.

### Synthesis of Block Copolymer

The synthesis of the block copolymers is carried out by the following procedure.

The block copolymers are obtained by synthesizing the homopolymer in the same manner as described above, followed by removing a part of the homopolymer and then adding a second monomer to the remaining homopolymer. For instance, to synthesize a block copolymer of CMS and MMA, CMS is added to potassium-naphthalene and the homopolymerization reaction is conducted for 24 hours. After removal of a part of the CMS homopolymer thus prepared, the remaining CMS homopolymer is further polymerized with MMA for an additional 4 hours to afford the block copolymer.

By comparing the molecular weights of the removed homopolymer and the resulting block copolymer, the molecular weight to which said second monomer makes contribution may be calculated.

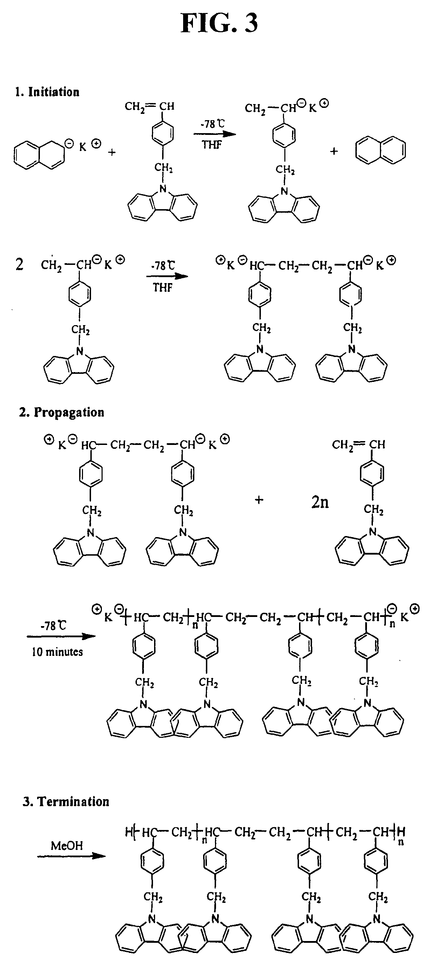

A reaction scheme of high molecular weight polymer of the present invention according to the anionic polymerization method can be seen in Fig. 3.

A better understanding of the present invention may be obtained in light of the following examples which are set forth to illustrate, but are not to be construed to limit the present invention.

### EXAMPLES 1-9

The synthesis results of homopolymer of CMS at -45 °C and -78 °C are shown in Table 1.

**TABLE 1**

| Run | Reaction Conc.(mmol) | | Time (min) | Temp.(°C) | Yield (%) | Molecular Weight (MW) | | MW Distribution |
|---|---|---|---|---|---|---|---|---|
| | K-Naph | CMS | | | | Calcd | Obsd | |
| 1 | 0.099 | 1.715 | 10 | -45 | 49.2 | 4,800 | 8,600 | 2.05 |
| 2 | 0.100 | 1.731 | 20 | -45 | 54.8 | 5,400 | 9,900 | 2.09 |
| 3 | 0.102 | 1.700 | 30 | -45 | 64.1 | 6,100 | 10,700 | 2.04 |
| 4 | 0.099 | 1.667 | 120 | -45 | 67.2 | 6,400 | 10,000 | 2.11 |
| 5 | 0.095 | 1.613 | 10 | -78 | 91.0 | 8,700 | 9,600 | 1.67 |
| 6 | 0.097 | 1.686 | 20 | -78 | 93.3 | 9,200 | 9,700 | 1.58 |
| 7 | 0.104 | 1.819 | 30 | -78 | 97.3 | 9,700 | 9,500 | 1.65 |
| 8 | 0.096 | 1.536 | 24h | -78 | 98.0 | 8,900 | 8,800 | 1.55 |
| 9 | 0.094 | 1.766 | 10 | -78 | 96.0 | 10,600 | 9,300 | 1.28 |

The Table 1 shows the properties of high molecular weight polymer synthesized from CMS monomer at different temperatures.

From the table, it can be seen that, at a high temperature of -45 °C, there is a wide difference between the calculated molecular weight and the observed molecular weight with a broad range of molecular weight distribution. Also, it is noted that the yield is significantly changed when the running time is extended from 10 minutes to 120 minutes. At high temperatures, some reactive terminals of high molecular weight polymer attack methylene group of CMS monomer, and are thus unavailable for further reaction, while the reactive terminals at other sites remain unreacted, and thus molecular weight distribution becomes broader. Meanwhile, it can be seen that such side-reactions are restrained at a low temperature of -78 °C so that the distribution of the molecular weight becomes narrow, whereby the calculated molecular weight is similar to the observed one.

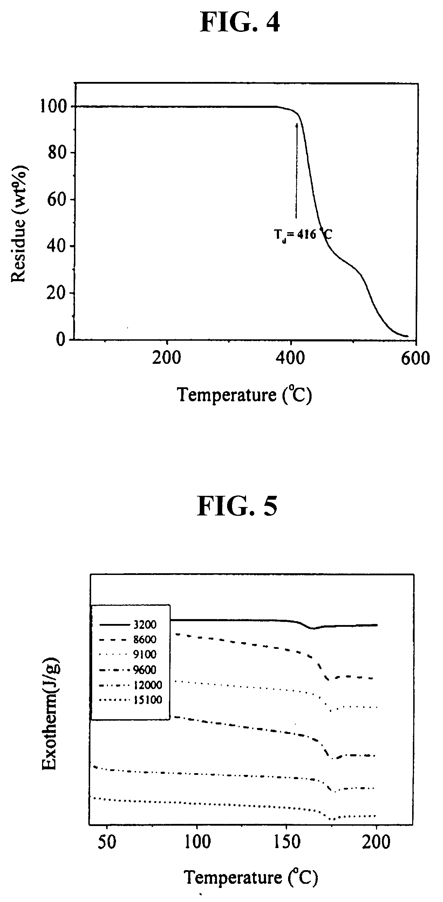

The thermal properties of synthesized homopolymer were measured by use of DSC (differential scanning calorimeter) and TGA (thermo gravimetric analysis). The results are shown in Figs. 4 and 5. By using TGA, the decomposition temperature of the high molecular weight polymer was found to be 416 °C, at which weight loss of 5 wt% occurs, as best seen in Fig. 4. The glass transition temperature was measured using DSC, and the temperature was seen to change from 159 °C to 173 °C, depending on the molecular weight, as seen in Fig. 5. The prepared homopolymer has a more thermally stable structure than polystyrene polymer, because of having much higher decomposition temperature and glass transition temperature than those (304 °C and 100 °C, respectively) of polystyrene polymer. When the molecular weight is 3,000 or more, the glass transition temperature exceeds 150 °C. When the molecular weight is 10,000 or more, the temperature becomes constant at 173 °C, though the glass transition temperature depends on the molecular weight.

Table 2 shows glass transition temperature varying with molecular weight.

**TABLE 2**

| Molecular weight | 3,200 | 8,600 | 9,100 | 9,600 | 12,000 | 15,000 |
|---|---|---|---|---|---|---|
| Glass Transition Temp. (°C) | 159 | 169 | 171 | 171 | 173 | 173 |

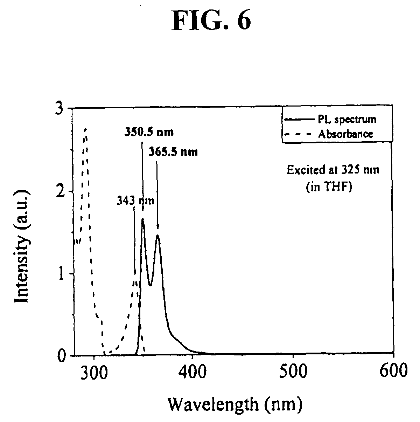

CMS synthesized in the present invention has luminescent properties, and is excited at a wavelength of 325 nm by use of He-Cd laser, whereby the luminescence peak is seen at 350.5 nm and 365.5 nm, as can be shown in Fig. 6.

### EXAMPLES 10-13

The synthesis results of copolymer of CMS and styrene or other monomer are given in Table 3.

**TABLE 3**

| Run | Reaction Con. (mmol) | | | | | | Time (min) | Temp. (°C) | Yield (%) | M.W. | | M.W. Dis. |
|---|---|---|---|---|---|---|---|---|---|---|---|---|
| | K-Naph | CMS | Styrene | DPE | MMA | CzMA | | | | calcd | obsd | |
| 10 | 0.091 | 2.030 | 5.552 | | | | 120 | -78 | 100 | 25,700 | 27,000 | 1.51 |
| 11 | 0.104 | 0.839 | 5.437 | | | | 120 | -78 | 100 | 16,000 | 17,000 | 1.47 |
| 12 | 0.130 | 2.153 | | 0.153 | 5.106 | | 300 | -78 | 98 | 17,500 | 20,000 | 1.48 |
| 13 | 0.111 | 2.158 | | 0.122 | | 2.129 | 300 | -78 | 100 | 22,000 | 23,000 | 1.48 |
| Example 10 : first added monomer-CMS, second added monomer-styrene | | | | | | | | | | | | |
| Example 11 : first added monomer-styrene, second added monomer-CMS | | | | | | | | | | | | |

From the result of table 3, it can be seen that, after the homopolymerization of CMS, the addition of the second monomer such as styrene, methylmethacylate (MMA) or 9-ethylcarbazollyl methacylate (CzMA) results in the synthesis of a block copolymer. This means that the addition of the second monomer to reactive terminals of CMS leads to continuous polymerization. From the synthesis of the block copolymer of CMS and styrene, it is confirmed that the block copolymer is synthesized regardless of addition order of CMS and styrene to the reaction. Accordingly, reactivity of CMS and styrene is assumed to be similar. Also, it is seen from the data of the block copolymers of CMS and MMA or CzMA that the reactivity of CMS is larger than that of MMA and CzMA.

In accordance with the method of the present invention, a homopolymer and a block copolymer can be synthesized by use of a solid styrene monomer containing carbazole.

The high molecular weight polymer of the present invention synthesized from carbazole-containing monomers with optical properties is advantageous in terms of various optical properties, limited molecular weight distribution, and improved thermal stability and solubility. Accordingly, the temperature is decreased on polymerization and thus side-reactions are minimized, so that the solid monomer can be synthesized according to the method of the present invention.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. A 4-mehylcarbazollylstyrene derivative containing carbazole as functional side group.

2. A method for synthesizing homopolymer, poly(4-(9-carbazollylmethylstyrene)), by use of 4-methylcarbazollyl styrene derivative of claim 1 as monomer.

3. The method as set forth in claim 2, wherein a reaction temperature is -78 °C.

4. The method as set forth in claim 2, wherein tetrahydrofuran is used as a solvent.

5. Poly(4-(9-carbazollylmethylstyrene)), homopolymer containing carbazole as functional side group, prepared by the method of claim 2.

6. A method for synthesizing a block copolymer of 4-methylcarbazollylstyrene derivative of claim 1 and styrene, MMA or CzMA.

7. The method as set forth in claim 6, wherein the reaction temperature is -78 °C.

8. The method as set forth in claim 6, wherein tetrahydrofuran is used as a solvent.

9. A block copolymer of 4-methylcarbazollylstyrene derivative containing carbazole as functional side group and styrene, MMA or CzMA, prepared by the method of claim 6.
